# EUROPEAN PATENT SPECIFICATION

(11) **EP 4 213 770 B1**
(45) Date of publication and mention of the grant of the patent: **29.10.2025**
(21) Application number: 21870143.1
(22) Date of filing: 15.09.2021
(51) Int. Cl.: A61F 2/30, A61F 2/42

(54) **TOTAL ARTHROPLASTY OF THE CARPOMETACARPAL JOINT**
TOTALE ARTHROPLASTIE DES KARPOMETAKARPALGELENKS
ARTHROPLASTIE TOTALE DE L'ARTICULATION CARPO-MÉTACARPIENNE

(30) Priority: 15.09.2020 US 202063078499 P
(43) Date of publication of application: 26.07.2023
(73) Proprietor: New York Society for the Relief of the Ruptured and Crippled, Maintaining the Hospital for Special Surgery, New York, NY 10021 (US)
(72) Inventor: HOTCHKISS, Robert, Riverside, Connecticut 06878 (US); DALUISKI, Aaron, New York, New York 10021 (US); WRIGHT, Timothy, New York, New York 10021 (US); QUEVEDO-GONZALEZ, Fernando, Greenwich, CT 06830 (US); LIPMAN, Joseph, New York, New York 10025 (US); OSEI, Daniel, New York, New York 10021 (US); LO, Darrick, New York 11226 (US); SARKAR, Ritvik, New York, New York 10021 (US)
(74) Representative: Studio Torta S.p.A.
(86) International application number: PCT/US2021/050486
(87) International publication number: WO 2022/060853

(56) References cited:
- WO-A1-95/09587
- FR-A1- 2 805 151
- US-A- 6 126 690
- US-A1- 2002 055 785
- US-A1- 2003 216 813
- US-A1- 2005 033 426
- US-A1- 2011 077 652
- US-A1- 2011 184 527
- US-A1- 2011 202 140
- US-A1- 2011 288 550
- US-A1- 2015 342 745
- US-A1- 2019 167 437
- US-B2- 8 105 388

## Description

### Technical Field

The present disclosure relates generally to prosthetic replacement joints and more particularly, relates to a prosthesis (implant) that is configured to replace a damaged carpometacarpal joint of the base of the thumb. The closest prior art is document WO 95/09587 Al, which defines the preamble of claim 1.

### Background

The human body has many bones and joints. One of the more important body parts is the hand. The hand is composed of many small bones called carpals, metacarpals and phalanges. The two bones of the lower arm, namely, the radius and the ulna, meet at the hand to form the wrist. The thumb is the most important single digit of the hand, positioned in opposition to the other digits to allow prehension - pinch, grasp and grip. Functionally, the most important joint of the thumb is the carpometacarpal (CMC) joint which is located at the very base of the thumb. The thumb carpometacarpal (CMC) joint, a biconcave-convex saddle joint, consists of the articulation between the thumb metacarpal and the trapezium carpal bone.

Both the shape of the joint and its position relative to the tip of the thumb, optimize the utility of hand function by permitting a wide range of positions of the thumb relative to the fingers. This freedom of position allows the human hand to accommodate a wide variety of objects of different shapes, sizes and weights to be securely held and accurately manipulated in space. The human thumb, and specifically, the CMC joint, is perhaps the most sophisticated version of the "opposable" thumb of all primates and is the foundation for both powerful grasp and fine motor skills.

Injury, ageing, osteoarthritis and rheumatoid arthritis are common causes of deterioration of the CMC joint. The early symptoms can begin with a subtle loss of pinch strength and an occasional sudden, episodic jolt of pain when grasping a doorknob or a toothbrush. This is usually due to a combination of instability and mild inflammation. Nonsurgical treatment is usually recommended at this stage.

However, with progression of the condition, the deformity and joint subluxation often becomes more pronounced as the cartilage thins. The pain becomes more constant and strength continues to deteriorate to the point where simple activities like writing, holding a glass or cup, or simple self-care are quite challenging. At this juncture, nonsurgical care is no longer effective, and patients seek relief of pain, improved alignment and stability. As with all joint replacements, restoration of the articulation with stability, requisite alignment and a smooth gliding surface offers an approximation of the pre-arthritic state and restoration of hand function and quality of life.

Since the thumb is so important to perform normal daily tasks, the instability and/or the subsequent loss of function of this important joint is troubling. This joint is considered unstable when it exhibits gross abnormal alignment, which is often combined with excessive and aberrant mobility. An unstable joint may, over time, become fixed and deformed. Instability of the CMC joint is often caused by osteoarthritis or rheumatoid arthritis. Osteoarthritis develops relatively frequently at the CMC joint of the thumb, often as a result of athletic injury. In a total arthroplasty, both surfaces of the joint are replaced. Thumb carpometacarpal (CMC) joint implants include a trapezium implant defining an articulating surface and a cooperating thumb metacarpal implant with a base portion of the thumb metacarpal defining an articulating surface. The thumb metacarpal base articulating surface is configured to articulate against the trapezium implant articulating surface. Most commonly, the metacarpal component includes a stemmed portion with the stem extending into the medullary canal of the metacarpal as a means of fixing the component to the surrounding bone. It will be appreciated that the terms "articulation surface" and "articular surface" and "articular region" can be used interchangeably and refer to the region/surface at which articulation occurs.

One of the deficiencies of traditional CMC joint prostheses (implants) is that the metacarpal component of the prosthesis loosens over time as fixation is lost between the stem and the surrounding bone of the metacarpal. In addition, traditional CMC implants often replace the biconcave-convex saddle joint with other geometries, like a ball-in-socket, that do not restore adequate function and often over constrain the joint surfaces. There is therefore a need to provide a CMC implant that overcomes these deficiencies as well as others.

### Summary

The present invention is defined in claim 1 and is directed to a prosthesis (implant) that is configured to replace the damaged carpometacarpal joint of the base of the thumb. The prosthesis comprises a metacarpal component that replaces the proximal articular surface of the thumb metacarpal and a trapezial component that replaces the distal articular surface of the trapezium and is configured to be secured to the trapezium bone. As described herein, one of the advantages of the present prosthesis is that it is intended for non-cemented fixation to both the thumb metacarpal and the trapezium and includes surfaces designed for bone ingrowth into the implant.

The articulation of the prosthesis is saddle-shaped (i.e., each of the metacarpal component and the trapezial component has a saddle-shaped articular region) and in one embodiment the articulation is UHMWPE (ultra-high molecular weight polyethylene) against CoCr or titanium alloy. Accordingly, in one embodiment, one of the metacarpal component and the trapezial component has a UHMWPE articulation, while the other of the metacarpal component and the trapezial component has a CoCr or titanium alloy articulation. If titanium is used, the surface can be nitrided to harden the surface to improve wear characteristics. The articulation can also be PEEK on UHMWPE or other suitable biocompatible materials.

The metacarpal component according to one embodiment can have a multi-piece construction that consists of a (Ti-6Al-4V alloy) stem with a baseplate and a saddle-shaped (UHMWPE) insert that is pre-assembled into the baseplate. It will be appreciated that the stem is integral to the baseplate and the combination of the baseplate and the insert can be considered to define a main body portion of the metacarpal component. The stem is inserted into the prepared cavity of the thumb metacarpal. The proximal surface of the stem preferably has an ingrowth surface that can be created via plasma spray, annealed beads, or 3D printing of the stem. The metacarpal component is symmetric in the radio-ulnar direction such that it can be used for both left and right hands. In another embodiment, the metacarpal component is formed as a single piece, monobloc construction and includes the stem and a main body portion that includes the saddle-shaped articular region with the stem protruding outwardly from the main body portion.

The trapezial component can be a single piece, monobloc saddle-shaped component. It is designed to resurface the distal saddle-shaped anatomy of the trapezium where the trapezium articulates with the thumb metacarpal. The component is symmetric in the radio-ulnar direction such that it can be used for both left and right hands. The fixation, according to one embodiment, consists of a central (cruciform) shaped peg that requires a pre-drilling operation before implantation, along with a plurality (e.g., two) spikes that are stiff enough to penetrate the cancellous bone without requiring pre-drilling. As discussed herein, the fixation, in one embodiment, is constructed to promote bone ingrowth and, more particularly, a surface of the fixation preferably has an ingrowth surface that can be created via plasma spray, annealed beads, or 3D printing of the fixation. In another embodiment, the trapezial component can have a multi-piece construction that consists of a (Ti-6Al-4V alloy) baseplate with integral fixation features described above and an (UHMWPE) insert having a saddle-shaped articular region that is preassembled into the baseplate. In this embodiment, the baseplate and the saddle-shaped articular region define the main body portion of the trapezial component and the fixation features protrude outwardly from the underside of the main body portion (i.e., the underside of the baseplate).

In one embodiment, the prosthesis for the CMC joint of a thumb thus includes a metacarpal component that has an articular surface and a stem configured for insertion into the medullary canal of a thumb metacarpal bone to secure the metacarpal component to the thumb metacarpal bone. The stem has a dorsal surface and an opposing volar surface. Each of the dorsal surface and the volar surface is defined by a proximal region and a distal region. The proximal region of the volar surface has a volar taper angle that is greater than the volar taper angle of the distal region of the volar surface and a dorsal taper angle of the proximal region of the dorsal surface that is less than the volar taper angle of the proximal region of the volar surface.

It will be understood that in the complete prosthesis, the trapezial component articulates with the metacarpal component, providing a gliding interface between the two components, restoring stable movement.

### Detailed Description of the Drawing Figures

Figs. 1A, 1B, and 2 are schematics of a human hand including a prosthesis (implant) that is configured to replace the damaged carpometacarpal joint of the base of the thumb;
Figs. 3A-3D illustrate a first saddle-shaped articulation region of one exemplary metacarpal component of the prosthesis and a second saddle-shaped articulation region of one exemplary trapezial component of the prosthesis;
Fig. 4 illustrates one exemplary stem of the metacarpal component;
Fig. 5A is a side elevation view of the metacarpal component;
Fig. 5B is one end view thereof;
Fig. 5C is another end view thereof;
Fig. 5D is a bottom plan view thereof;
Fig. 5E is a first cross-sectional taken along the line 1-1 of Fig. 5A;
Fig. 5F is a second cross-sectional of the stem of Fig. 5A;
Fig. 6A shows the fit of the stem in a representative metacarpal bone at three cross-sections, shown in Figs. 6B, 6C and 6D along the length of the stem;
Fig. 7 is a schematic showing a linear variation of stiffness of the stem due to a material with higher elastic modulus at the stem's proximal end and a material with lower elastic modulus at the stem's most distal end along with a density of the bone being depicted in grayscale with lighter tones defining higher bone densities;
Fig. 8 is a side perspective view of the stem illustrating a porous coating applied thereto;
Fig. 9A is a side elevation view of one exemplary trapezial component;
Fig. 9B is an end elevation view thereof;
Fig. 9C is a bottom plan view thereof;
Fig. 9D is a close-up of the area included in the circle in Fig. 9A;
Fig. 9E is a cross-sectional taken along the line 1-1 of Fig. 9A;
Fig. 9F is a cross-sectional view of a peg that forms part of the fixation features of the trapezial component;
Fig. 10A is a side elevation view of another exemplary trapezial component;
Fig. 10B is an end elevation view thereof;
Fig. 10C is a cross-sectional view taken along the line 1-1 of Fig. 10A;
Fig. 10D is a cross-sectional view of a peg that forms part of the fixation features of the trapezial component;
Fig. 10E is a bottom plan view thereof;
Fig. 11A is a bottom plan view of another exemplary trapezial component;
Fig. 11B is a bottom plan view of another exemplary trapezial component;
Fig. 11C is a bottom plan view of another exemplary trapezial component;
Fig. 11D is a bottom plan view of another exemplary trapezial component;
Fig. 12A is a top and side perspective view of a metacarpal component according to another embodiment;
Fig. 12B is a bottom and side perspective view the metacarpal component of Fig. 12A;
Fig. 12C is a side elevation view thereof;
Fig. 12D is an end elevation view thereof;
Fig. 12E is a bottom plan view thereof;
Fig. 13A is a top and side perspective view of another exemplary trapezial component;
Fig. 13B is a side elevation view thereof;
Fig. 13C is a cross-sectional view taken along the line 1-1 of Fig. 13B;
Fig. 13D is an end elevation view thereof;
Fig. 13E is a first bottom view;
Fig. 13F is a second bottom view;
Fig. 14A is a side elevation view another exemplary trapezial component;
Fig. 14B is a cross-sectional view taken along the line 1-1 of Fig. 14A;
Fig. 14C is an end elevation view thereof; and
Fig. 14D is a bottom view.

### Detailed Description of Certain Embodiments

The present device (e.g., prosthesis) is intended to resurface the damaged articular surfaces of the trapezium and the thumb metacarpal bone in patients with painful arthritis of the carpometacarpal joint of the thumb, with or without instability, and/or limited motion, as a result of rheumatoid arthritis, traumatic arthritism osteoarthritis, or post-fracture deformation of the articulating bones.

### Prosthesis 100

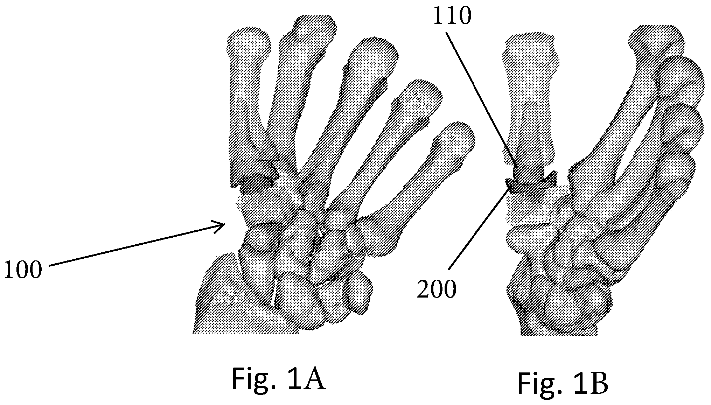

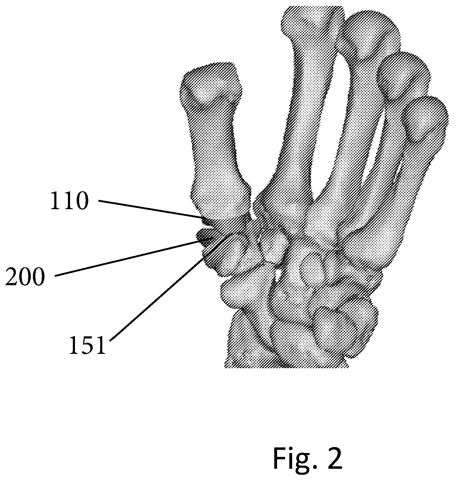

Now turning to Figs. 1A-11D, one exemplary prosthesis (implant) 100 is illustrated and is configured to replace the damaged carpometacarpal joint of the base of the thumb as shown in Figs. 1A, 1B, and 2. The prosthesis 100 comprises a metacarpal component 110 that replaces the proximal articular surface of the thumb metacarpal and a trapezial component 200 that replaces the distal articular surface of the trapezium. As described herein, one of the advantages of the present prosthesis 100 is that it is intended for non-cemented fixation to both the thumb metacarpal and the trapezium and includes surfaces designed for bone ingrowth. Each of the metacarpal component 110 and the trapezial component 200 is described in detail below.

### Articular Surfaces of the Prosthesis 100

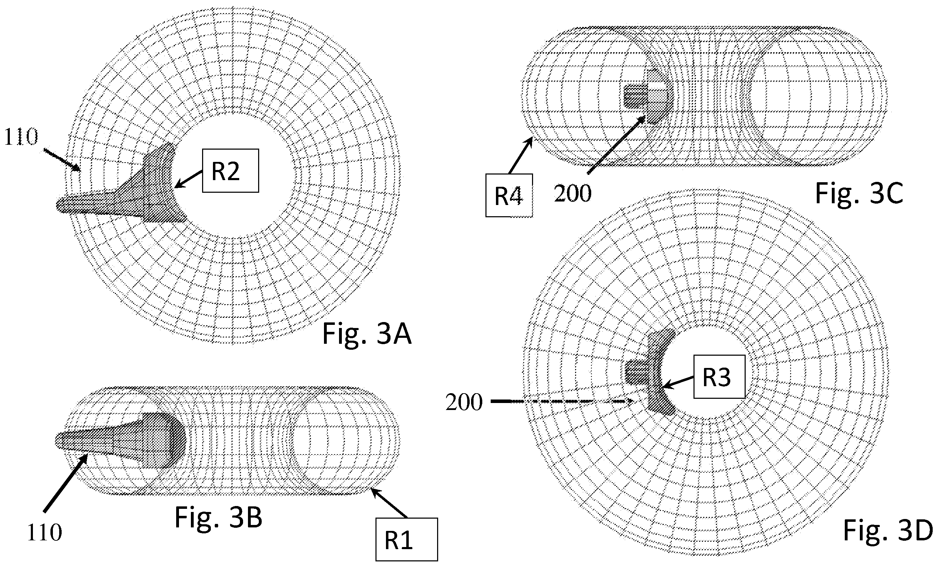

As described herein, the prosthesis 100 is designed to replace both the proximal articular surface of the thumb metacarpal and the distal articular surface of the trapezium and, therefore, the metacarpal component 110 and the trapezial component 200 are designed to articulate with respect to one another as in a healthy carpometacarpal joint. More specifically, the articulation of the prosthesis 100 is saddle-shaped and is composed of a toroid-on-toroid articulation. The metacarpal component 110 thus has a first saddle-shaped articulation region and the trapezial component 200 has a complementary second saddle-shaped articulation region that articulates with the first saddle-shaped articulation region. As shown in Fig. 3, the first saddle-shaped articulation region of the metacarpal component 110 is defined by a first radius (R1) in a radio-ulnar direction and by a second radius (R2) in a dorso-volar direction. Together, R1 and R2 define the first saddle-shaped articulation region of the prosthesis 100. As also shown in Fig. 3, the second saddle-shaped articulation region of the trapezial component 200 is defined by a third radius (R3) in the radio-ulnar direction and by a fourth radius (R4) in the dorso-volar direction. Together, R3 and R4 define the second saddle-shaped articulation region of the prosthesis 100.

In accordance with one embodiment of the prosthesis, a ratio of R4/R2 is between 80% to 96% and a ratio of R1/R3 is also between 80% to 96%. For example, in one implementation, R1=10 mm, R2=14.5 mm, R3=10.6 mm and R4=13.9 mm. The above ratio allows for axial rotation of the components 110, 200 relative to one another yet prevents edge loading of the articulation. It will also be appreciated that the metacarpal component 110 and the trapezial component 200 can be manufactured in different sizes and thus, can be offered as multiple size components 110, 200 to fit the variation in boney anatomy. However, the toroidal radii of the surfaces will stay the same across sizes to allow for the ability to match any size metacarpal component 110 with any trapezial component 200. In other words, a kit can be provided that includes multiple metacarpal components 110 and multiple trapezial components 200, and then the surgeon can select the proper components 110, 200 based on the anatomy of the patient.

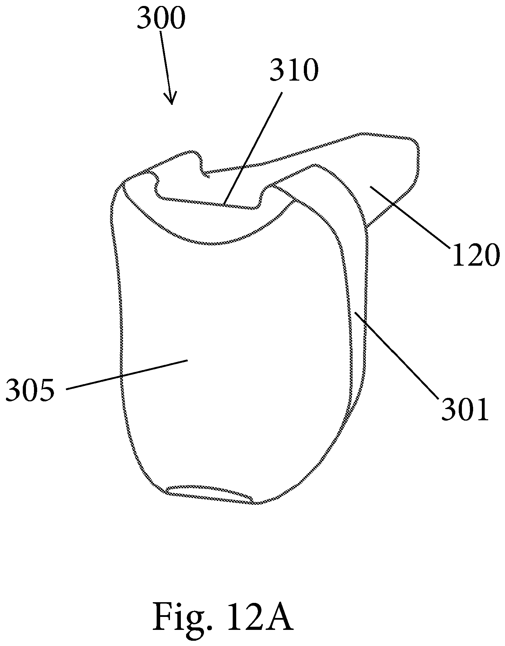

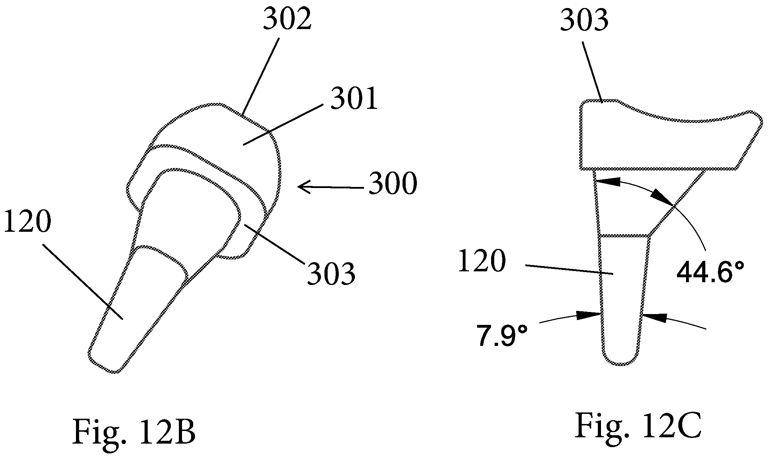

It will therefore be appreciated that in one embodiment, the distal (metallic) surface of the trapezial component articulates with the UHMWPE bearing of the first metacarpal component (Figs. 1A-11D), while in another embodiment, the distal UHMWPE bearing of the trapezial component articulates with the complementary (metallic) surface of the first metacarpal component as shown in Figs. 12A to 14D.

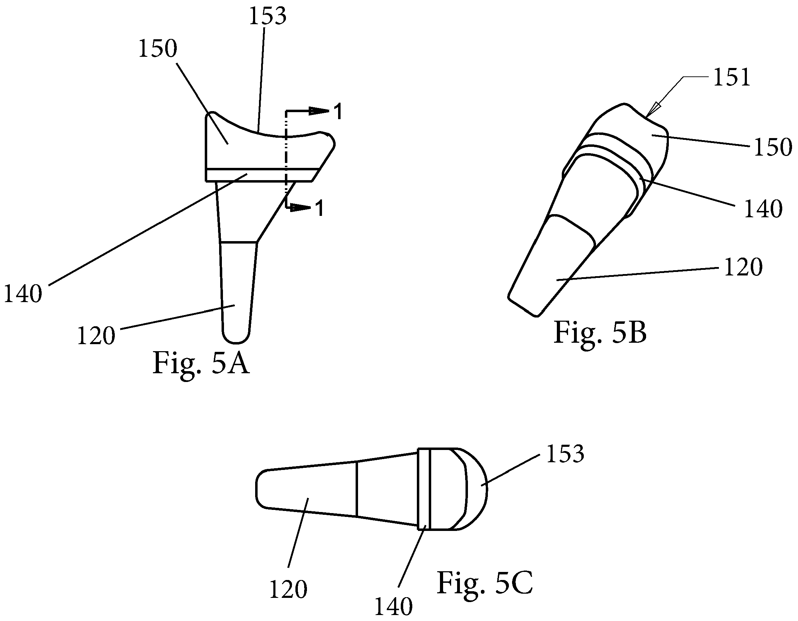

The articulation region is symmetric in both the dorso-volar direction and the radio-ulnar direction. As will be described in more detail herein, the distal surface of the trapezial component 200 articulates with a proximal bearing (insert 150) of the metacarpal component 110. The insert 150 can be formed of any number of materials that are suitable for its intended use as an articular surface, and in one embodiment, the insert 150 is formed of UHMWPE. The peaks of the saddle surface on the radial and ulnar ends of the trapezial component 200 mimic the native anatomy of a healthy trapezium. The metacarpal component 110 articulation orients the saddle perpendicular to the trapezial component 200. Therefore, the peaks of the saddle surface are on the dorsal and volar sides of the prosthesis (implant) 100. As shown in Fig. 5B, the articular surface of the insert 150 includes a volar lip that resists dorsal subluxation of the thumb. In accordance with one aspect of the present disclosure, the volar lip includes a concave cut-out. This concave cut-out 151 is purposely formed along the volar lip so as to reduce the chance of impingement on the trapezium when flexing the thumb (See, Figs. 2 and 5B). In other words, the concave cut-out 151 reduces or eliminates bone interference. Furthermore, the volar lip cutout has the additional advantage of facilitating the reduction of the joint during implantation.

### Metacarpal Component 110

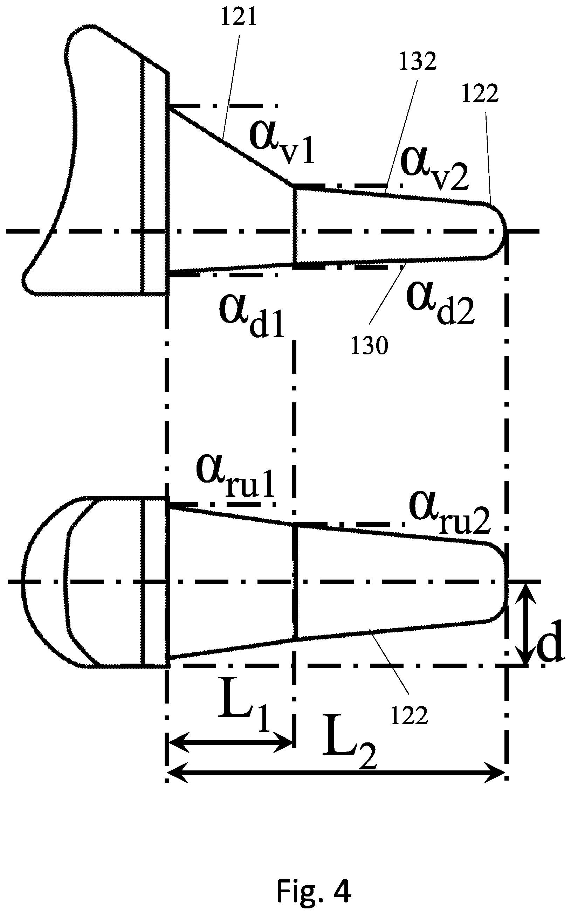

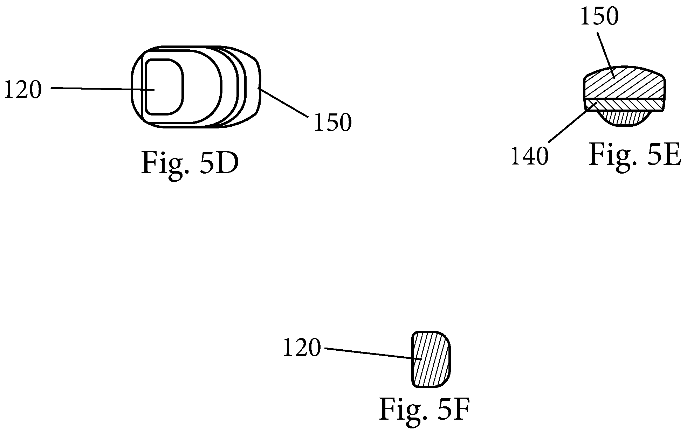

As shown in Figs. 4 and 5A-5F, the metacarpal component 110 includes a stem 120 that is inserted into a prepared cavity of the thumb metacarpal, a baseplate 140, and the insert 150 (e.g., a UHMWPE insert) that articulates with the trapezial component 200. As shown in Fig. 5, these three regions define the metacarpal component 110 and the combination of the baseplate 140 and insert 150 can be thought of as defining a main body portion of the metacarpal component 110 from which the stem 120 extends outwardly. It will be appreciated that the baseplate 140 and the stem 120 are a single integral structure. The baseplate 140 is thus joined to (interfaces with) one end of the stem 120 and is configured to receive and be coupled to the insert 150 along the surface opposite to the stem 120. The articular surface of the metacarpal component 110 is generally indicated at 153 and forms an exposed outer surface of the insert 150.

The prosthesis 100 is symmetric in the radio-ulnar direction, to allow for interchangeability between left and right hands. The shape and material properties of the stem 120 have been optimized to minimize: 1) the relative motion between the prosthesis (implant) 100 and the bone, 2) the risk of bone failure, and 3) the stress shielding of bone. The axis of the stem 120 is located at a distance d from the dorsal edge of the implant's baseplate to maximize coverage of the resected bone surface by the baseplate 140 (See, Fig. 4).

The stem 120 is specifically constructed so as to be inserted and held within the prepared cavity of the thumb metacarpal without the use of cement or other bonding agents. In addition, as described herein, the stem 120 is specifically configured and constructed such that it overcomes the deficiencies associated with conventional stem design. More specifically, conventional stems that were inserted into a prepared cavity (medullary canal) of the thumb metacarpal were prone to loosening over time and would fail. The present stem 120 addresses and overcomes these issues by having an optimized configuration that is specifically designed to fit and be anchored by boney ingrowth within the prepared cavity (medullary canal) of the metacarpal. In other words, the shape of the stem 120 is designed in view of the anatomical landscape of the prepared cavity and the stiffness of the stem 120 is regionally chosen to optimize the load transfer to the bone.

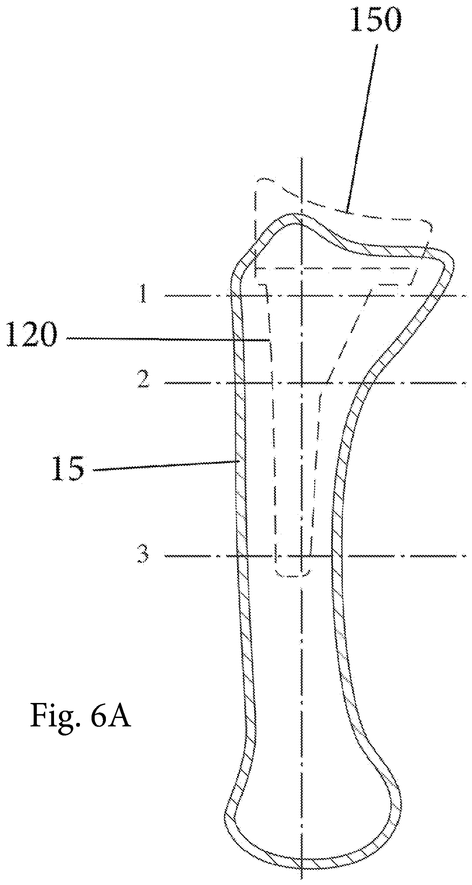

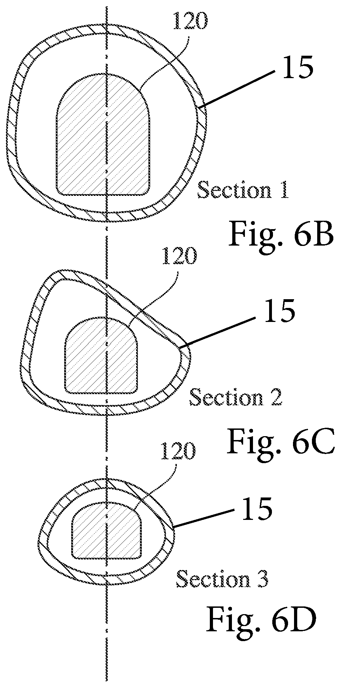

The shape of the stem 120 can be thought of as being generally defined by three cross-sections: proximal, intermediate, and distal as shown in Figs. 6A-6D. In Fig. 6A, the three cross-sections are taken along the lines 1, 2 and 3, respectively (see below discussion). As a result of this construction, the stem 120 can be divided into a proximal region 121 and a distal region 122. The proximal region 121 extends to and includes the proximal end of the stem and the distal region 122 extends to and includes the distal end of the stem 120. It will be appreciated that the longitudinal length of the proximal region 121 and the distal region 122 is not necessarily the same and in fact, in one embodiment, the two lengths are different. The stem 120 further is defined by a dorsal surface 130 and an opposing volar surface 132. It will be appreciated that the dorsal surface 130 and the volar surface 132 extend the entire length of the stem 120 and thus, both the proximal region 121 and the distal region 122 can be described in terms of the characteristics and features of both the dorsal surface 130 and the volar surface 132. In addition, as described below, the stem 120 also has a tapered construction, in a longitudinal direction, in that both the dorsal surface 130 and the volar surface 132 are tapered.

As shown in Figs. 6A-6D, the stem 120 can be constructed such that a cross-section of the stem 120 that is taken perpendicular to a longitudinal axis of the stem 120, the dorsal surface 130 is at least substantially flat (e.g., is flat), while the volar surface 132 is curved. As shown, the radius of curvature of the volar surface 132 changes along the longitudinal length of the stem 120 and more specifically, each of the proximal region 121, the intermediate region and the distal region 122 has a different radius of curvature. The changing radius of curvature exists along the longitudinal length of the stem 120 and this includes both the proximal region 121 and the distal region 122 as shown in Figs. 6A-6D.

The stem 120 has a tapered shape in the dorso-volar and radio-ulnar directions. Stem cross-sections taken transversely along its length preferably include rounded corners and can be thought of as being generally rectangular shaped (See, Figs. 6B-6D). In the radio-ulnar direction, the cross-sections are symmetric to allow for interchangeability between left and right hands. The rounding radius for the volar corners is greater than for the dorsal corners, resulting in a cross section that is flatter dorsally than volarly as shown in Figs. 6A-6D.

The tapered construction of the stem 120 is best shown in Fig. 4 and can be defined by a series of taper angles. More specifically, the taper of the stem 120 is different in the proximal region 121 than in the distal region 122. The taper angles of the stem 120 in the proximal region 121 (αᵥ₁, α_{d1}, αᵣᵤ₁) are larger than for the distal region 122 (αᵥ₂, α_{d2}, αᵣᵤ₂). The dorsal taper angles (α_{d1}, α_{d2}) are small to create the relatively flat surface of the stem 120 that runs parallel to the dorsal flat of the thumb metacarpal in the dorso-volar plane. On the volar side, the stem 120 has a steep taper angle proximally (αᵥ₁) that follows the proximal-volar cortex. Accordingly, the distal region 122 is straighter than the proximal region 121.

The shape of the stem 120, with larger taper angles proximally than distally, a relatively flat dorsal surface 130, and a steep volar surface 132 follows the shape of the thumb metacarpal canal (the prepared cavity) and allows the stem 120 to slide along the dorsal edge of the thumb metacarpal and achieve a wedge-type fit against the volar cortex (Figs. 6A-6D).

Figs. 6A-6D thus show the fit of the stem 120 in a representative metacarpal bone 15 at three cross-sections along the length of the stem 120. Fig. 6B is a cross-section taken along the line 1 of Fig. 6A; Fig. 6C is a cross-section taken along the line 2 of Fig. 6A; and Fig. 6C is a cross-section taken along the line 3 of Fig. 6A.

This combination of flat dorsal and steep volar surfaces allows the stem 120 to slide along the dorsal edge of the thumb metacarpal and achieve a wedge-type fit against the volar cortex. The values of the radio-ulnar taper angles (αᵣᵤ₁, αᵣᵤ₂) are in between those for the dorsal and volar taper angles. The aforementioned tapered construction is specifically designed in view of the implant location and more particularly, in view of the shape of the prepared cavity (inner canal) of the thumb metacarpal. This tapered construction, as well as the flat dorsal surface and curved volar surface, allows the stem 120 to be wedged into the surrounding anatomical landscape of the implant site.

In one embodiment, the total length of the stem 120 is between 15 mm and 23 mm. The proximal region 121 can extend between 30% and 45% of the total length of the stem 120. In the proximal region 121, the volar taper angle (αᵥ₁) can be between 40° and 52°, the dorsal taper angle (α_{d1}) can be between 3° and 6°, and the radio-ulnar taper angle (αᵣᵤ₁) can be between 8° and 24°. In the distal region 122, the volar taper angle (αᵥ₂) can be between 3° and 9°, the dorsal taper angle (α_{d2}) can be less than or equal to 3°, and the radio-ulnar taper angle (αᵣᵤ₂) can be between 4° and 6°.

### Material Characteristics of Stem 120

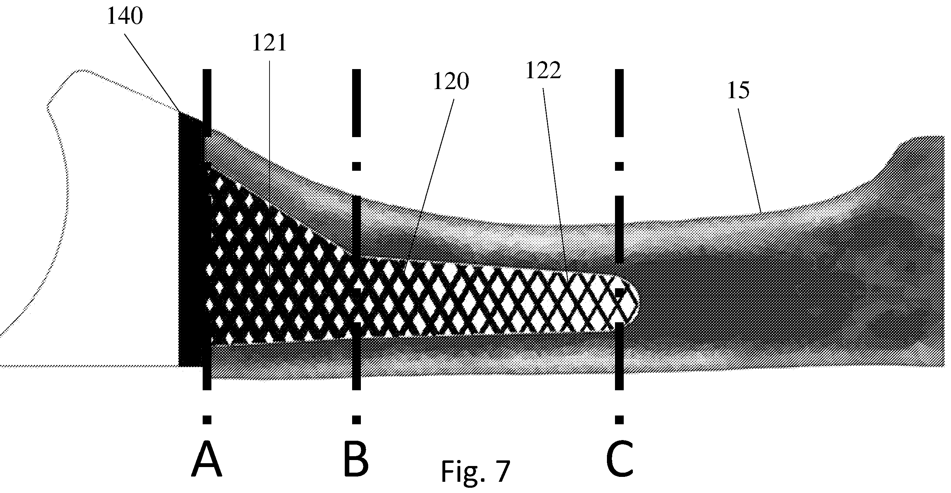

The stem 120 can be formed of any number of suitable materials including biocompatible metals. In one embodiment, the stem 120 can be made of completely solid Ti-6Al-4V alloy, with an elastic modulus of approximately 120 GPa. Alternatively, the stiffness of the stem 120 can be non-homogeneous by controlling the porosity, and thus the elastic modulus, of the stem material (e.g., a biocompatible material). As an example, the exemplary stem 120 illustrated in Fig. 7 has a linear variation of the stiffness due to a material with higher elastic modulus at the stem's proximal end and a material with lower elastic modulus at the stem's most distal end (i.e., the material's elastic modulus can progressively decrease along the length of the stem 120 in the direction from the proximal end to the distal end of the stem 120). In addition, the tapered shape of the stem 120 also provides for varying in stiffness along its length. Fig. 7 also shows the bone density (in grayscale with higher bone densities being depicted in lighter grayscale tones) which varies from inner regions of the bone to the outer region of the bone. One optimized stem 120 has a material with a proximal modulus of 80 GPa and a distal modulus of 49 GPa (See, Fig. 7). Applicant has discovered computational evidence that supports that this varying stiffness stem is less sensitive to variations in implant position than its stiffer counterpart made of solid Ti-6Al-4V alloy. In other words, even if the varying stiffness stem 120 is positioned less than perfectly (within the metacarpal canal), the micro-motion between the stem 120 and bone (thumb metacarpal), the bone failure, and the stress shielding remain within acceptable limits.

Fig. 7 includes reference lines A, B and C that identify certain points (reference areas) along the stem 120. Reference line A identifies the proximal end of the stem 120, reference line B identifies an intermediate location of the stem and more particularly, identifies the location at which the stem undergoes a more dramatic outward taper, and reference line C identifies the distal end of the stem 120. The modulus at or about reference line A can be between 50-120 GPa, modulus at or about reference line B can be between 34-119 GPa; and the modulus at or about reference line C can be between 12-118 GPa. In one exemplary embodiment, the modulus at or about reference line A is 80 GPa, the modulus at or about reference line B is 69 GPa and the modulus at or about reference line C is 49 GPa. It will be appreciated that the modulus at or about reference line B is a linear interpolation between the modulus values at reference lines A and C. In the illustrated embodiment, the distance A-C (total length) is 17.2 mm and the distance A-B is 6.4 mm. In one embodiment, the elastic modulus at the distal end (reference line C) is between 15% and 98% of the value of the proximal elastic modulus (reference line A). As an example, one preferred stem has a proximal elastic modulus of 80 GPa and a distal elastic modulus of 61% of the proximal elastic modulus (i.e., 49 GPa). In another embodiment, the distal elastic modulus is between 50% and 70% of the proximal elastic modulus.

The stem 120 can be produced by any number of suitable manufacturing techniques, including but not limited to, additive manufacturing (3D printing) and can be made of solid Ti-6Al-4V, highly porous Ti-6Al-4V alloy, or a combination of porous and solid portions. The elastic modulus of highly porous Ti-6Al-4V is a function of the geometry of the pores and the porosity and can therefore be tailored to specific values. With 3D printing, the porosity can be homogeneous or graded throughout the stem in a controlled manner, resulting in a tailored non-homogeneous distribution of the stem's material properties. As an example, the stem 120 in Fig. 7 can consist of porous Ti-6Al-4V that is denser proximally and less dense distally, to achieve the target variation of the material properties and consequently of the stem's stiffness. A stem with reduced and homogeneous stiffness can be obtained with porous Ti-6Al-4V with uniform density.

### Stem Porosity

The stem 120 consisting of a combination of solid and porous portions can have said solid and porous portions arranged radially. The porous portions can have homogeneous or non-homogeneous porosity, resulting in homogeneous or non-homogeneous material properties. In one embodiment, the solid portion is located central to the stem 120, as a solid core surrounded by a porous layer. The relative thickness of the solid and porous layers controls the stiffness of the stem 120. Therefore, the relative thickness of the solid and porous layers can be chosen throughout the length of the stem 120 to achieve a stem with a variation of the stiffness equivalent to the one shown on Fig. 7. The thickness of the solid core can range from zero, resulting in a fully porous stem, to the total thickness of the stem, resulting in a fully solid stem 120.

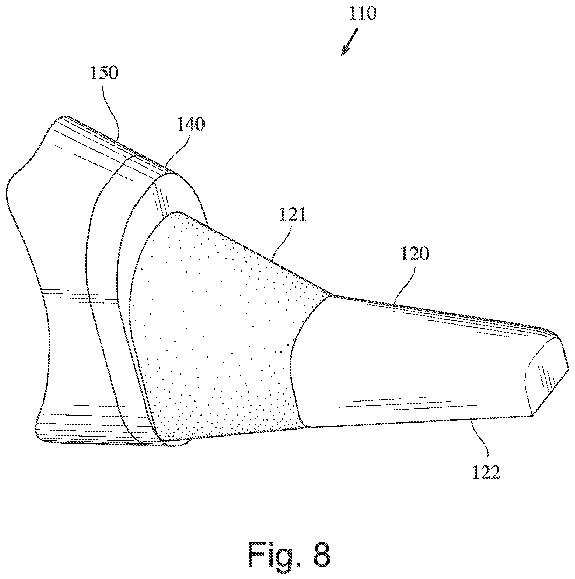

In addition, the outermost surface of the stem 120 can be made solid to avoid ingrowth between the highly porous material and the bone. The outer solid surface can partially or completely cover the stem 120. The outer solid surface can be treated partially (e.g., the most distal 55%) or in its entirety by conventional processing methods (e.g., polishing) to reduce the friction or the osseointegration properties. Ideally, the distal region 122 of the stem 120 is smooth to reduce the amount of bone that would have to be removed upon revision. In one preferred embodiment, only the proximal 30-45%, by length, of the stem 120 should have an ingrowth surface that corresponds with the more angled wedge portion of the stem (See, Fig. 8). In other words, the outer surface area of the stem 120 in the proximal region 121 is located within the wedge-like portion of the thumb metacarpal cavity (inner canal). This is the area in which bone ingrowth is desired, while the distal region 122 of the stem 120 is free of porosity. It is not desired to have excessive ingrowth surfaces (along the length of the stem 120) since this would require additional and likely excessive bone removal upon revision.

Accordingly, in Fig. 8, the proximal region 121 is shown to have a porous surface, while the distal region 122 has a polished surface.

Alternatively, if more ingrowth is required, the dorsal surface of the stem 120, or even the entire stem 120 can have an ingrowth surface.

### Metacarpal Component 300

Figs. 12A-12E illustrate another exemplary metacarpal component 300 that is intended to be used in combination with one of the trapezial components disclosed herein to form the prosthesis. The metacarpal component 300 shares a number of similarities with the metacarpal component 110 as discussed herein and therefore, the main differences between the two embodiments are highlighted below.

Unlike the multi-piece metacarpal component 110, the metacarpal component 300 is formed as a single-piece, monobloc component. While having a monobloc form, the metacarpal component 300, like the metacarpal component 110, has separate defined regions or sections and in particular, the metacarpal component 300 has a main body portion 301 that includes an underside 303, from which the stem 120 extends outwardly, and the articular surface 305, which articulates with the trapezial component. In other words, the stem 120 connects to (interfaces with) the main body portion 301 at the underside 303.

As in the previous embodiment, the stem 120 of the metacarpal component 300 is inserted into the prepared cavity of the first metacarpal and the main body portion 301 includes the saddle-shaped articular region (articular surface) which is generally identified at 305 in Fig. 12A. The articular surface 305 preferably has the properties discussed herein with respect to the saddle-shaped articular region discussed with reference to the metacarpal component 110. The main body portion 301 at the center of the saddle can be approximately 4.5 mm, corresponding to a minimal resection of the first metacarpal to preserve as much bone stock as possible while creating an optimal tension in the soft tissues.

The proximal surface of the stem 120 and the underside 303 of the main body portion 301 can have an ingrowth surface that can be a plasma spray, annealed beads, or created via 3D printing of the stem 120.

The stem 120 of the metacarpal component 300 can thus have the same construction and dimensions as the stem 120 of the metacarpal component 110 and therefore, have the same properties discussed herein with respect to the metacarpal component 110, including the varying elastic modulus.

The metacarpal component 300 can be formed of any number of suitable materials including those disclosed herein. For example, the metacarpal component 300 can be made of medical grade titanium alloy (Ti-6Al-4V) or cobalt-chrome alloy.

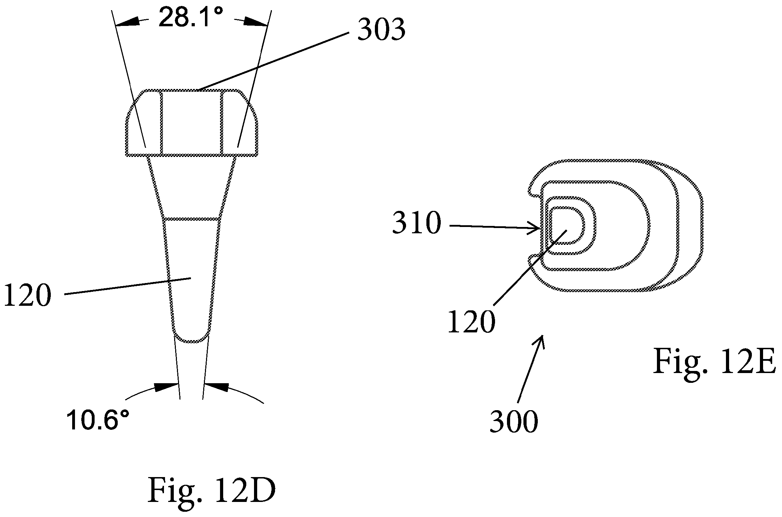

In accordance with one aspect of the present disclosure, the volar lip of the metacarpal component 300 includes a volar lip cut-out 302 much like the concave cut-out 151. This volar lip cut-out 302 is purposely formed along the volar lip so as to reduce the chance of impingement on the trapezium when flexing the thumb (See, Fig. 2). In other words, the volar lip cut-out 302 reduces or eliminates implant-bone interference. Furthermore, the volar lip cutout has the additional advantage of facilitating the reduction of the joint during implantation. The dorsal lip of the metacarpal component 300 resists volar subluxation of the thumb. A cut-out 303 on this dorsal lip reduces chance of impingement on the trapezium when extending the thumb (Figs. 12C and 12D).

In addition, the metacarpal component 300 has a dorsal access cut-out 310 which represents a notch formed along the dorsal edge (Figs. 12A and 12E). More specifically, the main body portion of the metacarpal component 300 has a cut-out along the dorsal edge that provides access to the flat dorsal surface of the stem 120. The cut-out 310 extends all the way through the main body portion and to the stem surface. It is approximately 6 mm wide to allow insertion of a chisel or osteotome to remove bone that has ingrown into the dorsal surface of the stem 120 to aid potential revision operations. The articular surface can be nitrided to improve the wear resistance against the articular region (UHMWPE material or insert) of the trapezial component.

As with the previous embodiment, the metacarpal component 300 is symmetric in the radio-ulnar direction to allow for interchangeability between left and right hands. The shape and material properties of the stem have been optimized to minimize: 1) the relative motion between the implant and the bone, 2) the risk of bone failure, and 3) the stress shielding of bone. The axis of the stem is located at a prescribed distance from the dorsal edge of the implant's main body portion to maximize coverage of the resected bone surface by the main body.

### Trapezial Component

As mentioned, the trapezial component is the second component of the prosthesis (implant) 100.

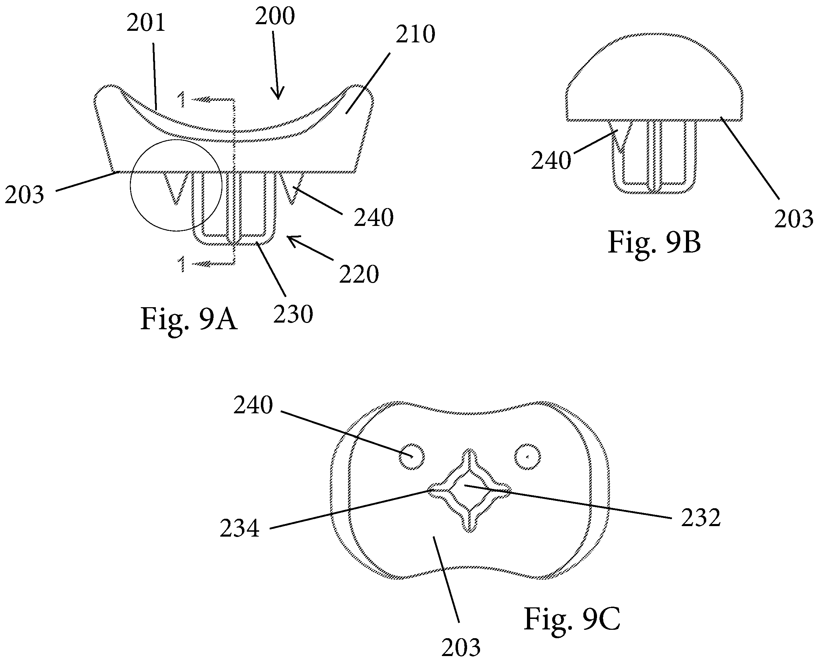

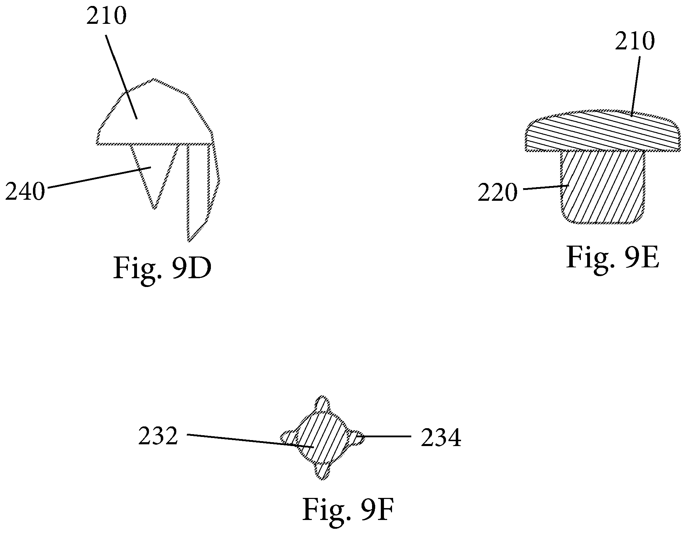

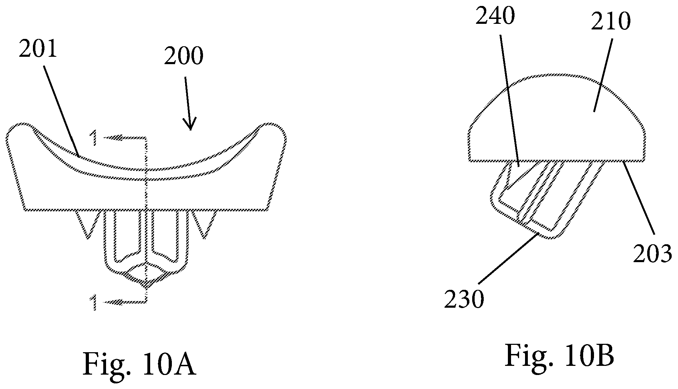

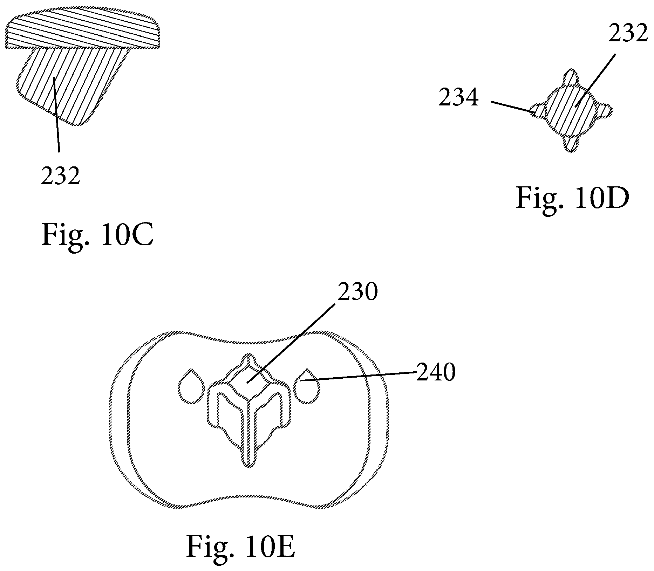

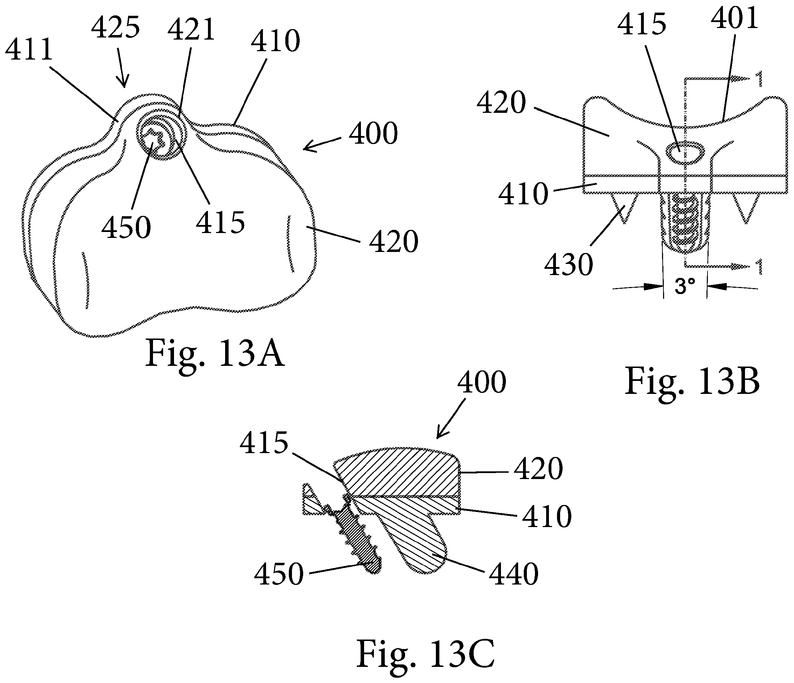

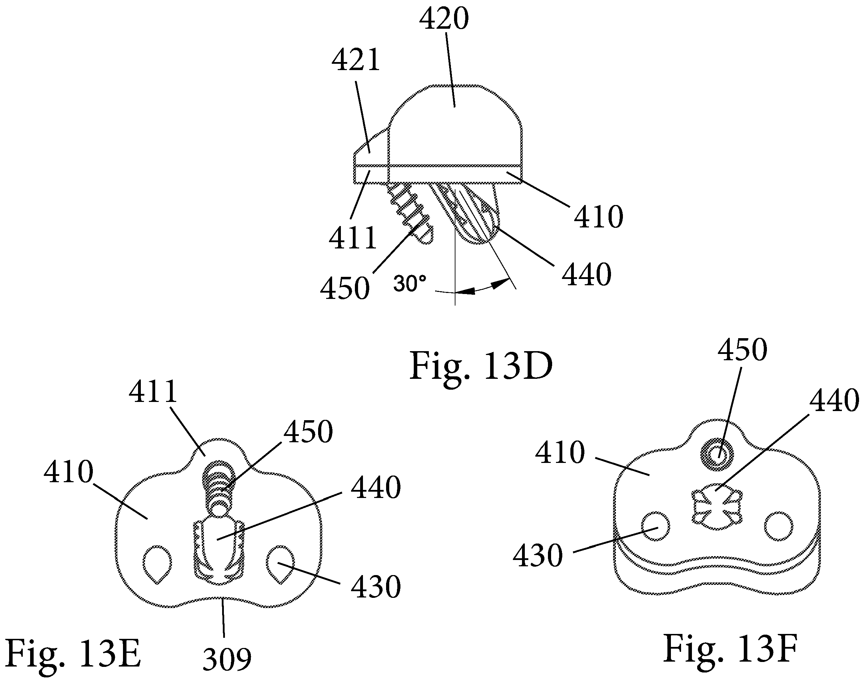

Much like the two different embodiments of the metacarpal component described above, the trapezial component can be formed either as a single-piece, monobloc component (Figs 9A to 9D and 10A to 10D) or can be formed as a multi-piece component (Figs. 13A to 13F and Figs. 12A to 14D). Each of these embodiments is discussed below.

### Trapezial component 200 (Figs. 9A to 9F)

As mentioned, in one embodiment, the trapezial component 200 can be a monobloc component made of medical grade titanium alloy (Ti-6Al-4V) or cobalt-chrome alloy. However, it will be appreciated that the trapezial component 200 can be formed of other suitable materials. The trapezial component 200 is designed to resurface the distal saddle-shaped anatomy of the trapezium where the trapezium articulates with the thumb metacarpal. If the articular surface of the trapezial component 200, generally indicated at 201, is made from titanium alloy, its articulating (articular) surface can be nitrided to improve the wear resistance against the UHMWPE of the metacarpal component. The trapezial component 200 is symmetric in the radio-ulnar direction such that a given component 200 can be used for both left and right hands.

The trapezial component 200 can be thought of as having two distinct regions, namely, a main body portion 210 located distally, that includes the articular surface 201 that faces the CMC joint along a first surface, and a fixation region 220, located proximally, that extends outwardly from the main body portion 210 and away from the articulation region (articular surface 201) into the native trapezium. As shown in the figures, the main body portion 210 includes a second surface that is opposite the first surface (the articular surface 201) and can be considered to be underside 203 of the main body portion 210. It will also be understood that the articular surface 201 is the surface that has a saddle shape that complements the saddle-shaped articular surface of the metacarpal component. The fixation region 220 interfaces with the underside 203 of the main body portion 210. As illustrated, the fixation region 220 extends outwardly from the underside of the main body portion 210; however, it will be appreciated that the fixation region 220 is integral to the main body portion 210 in that the two are formed as a single piece of material.

The trapezial component 200 can be manufactured from biocompatible metals or plastics (as well as any other suitable materials) using traditional subtractive manufacturing techniques or other suitable techniques. The trapezial component 200 can also be manufactured using additive manufacturing techniques. The fixation region 220 and the underside 203 of the main body portion 210 can be formed of a porous additively manufactured structure or can be coated with a porous ingrowth surface. This would allow bone ingrowth into the fixation features, described below, and the main body portion 210 for better long-term stability of the trapezial component 200.

The radial and ulnar edges can be angled away from the center of the prosthesis 100 such that the proximal edge of the profile is shorter than the distal edge. This allows a larger surface area for articulation while reducing the proximal footprint of the prosthesis 100. Alternatively, the radial and ulnar edges can straight edges, The proximal surface is planar and rests on the resection of the native trapezium bone. The footprint has a generally rectangular shape, a dorso-volar width less than the radio-ulnar width, rounded corners, and becomes thinner in the dorso-volar direction near the center of the articulation. This shape matches the general contour of the resected surface to minimize implant overhang. The reduced dorso-volar width also increases the range of motion of the carpometacarpal joint in flexion and extension.

Similar to the metacarpal component 110, the trapezial component 200 preferably comes in multiple sizes to better match the variation in patient anatomy. Thus, a kit can be provided in which there are different sized metacarpal components 110 and different sized trapezial components 200 for selection by the surgeon based on patient anatomy.

The footprint of the trapezial component 200 in one embodiment can extend 13-15 mm end to end in the radio-ulnar direction, 10-11 mm in the dorso-volar direction at the widest part, and 8-10 mm in the dorso-volar direction at the thinnest part as shown in Figs. 9A-9F. Surgeons choose the desired size of the device based on the footprint of the proximal surface. The size is chosen to maximize the footprint area on the resected surface while minimizing overhang of the implant (prosthesis 100).

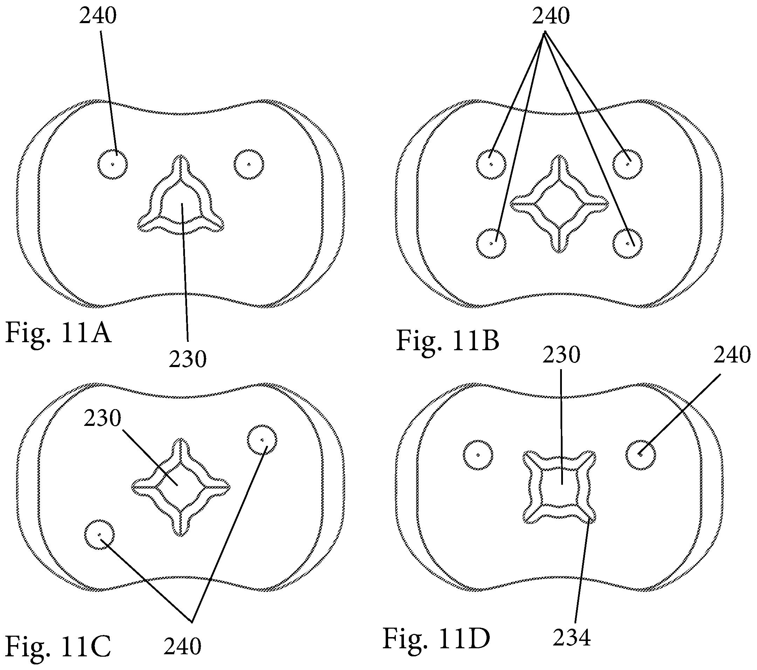

As mentioned, the trapezial component 200 has the fixation region 220 which serves as a means for anchoring the trapezial component 200 into the trapezium preferably without the use of any bonding agents (cement). The fixation region 220 includes one or more fixation features which, as shown, are integral with the main body portion 210. More specifically, the primary fixation feature of the trapezial component 200 can be a single peg 230 positioned at (or close to) the center of the underside 203 of the main body portion 210. As shown in Figs. 9A-9F, the peg 230 can have a generally cruciform cross section with a circular center portion 232 and that are arranged circumferentially around the center portion 232 and extend radially outward therefrom. The spacing between the radial flutes 234 can be uniform, as shown, or can be non-uniform. The radial flutes 234 are designed to engage bone and prevent rotation of peg 230 within the bone (trapezium). It will be appreciated that the cruciform shape of the peg 230 is an example of a four flute construction; however, the peg 230 can include less than four flutes 234 or more than four flutes 234. For example, Figs. 11A-11D shows a three flute example.

**In** one embodiment, the peg 230 has a cylindrical core with a diameter of 3-4 mm and a length of 3-5 mm from the underside 203 of the main body portion 210 as shown in Figs. 9A-9F. The peg 230 is sized to resist flexion-extension and abduction-adduction moments on the trapezial implant during activities of daily living.

As shown in Figs. 9A-9F, the peg 230 can be oriented to extend perpendicular to the underside 203 of the main body portion 210, or it can be angled by as much as 40° from the proximal-distal axis such that the peg 230 is biased in a volar direction as it extends away from the underside 203 of the main body portion 210 as shown in Figs. 10A-10D. Angling the peg 230 allows for easier insertion into the bone intraoperatively while maintaining resistance to bending moments applied across the CMC joint. When the peg 230 is angled, the introduction of any instrument used to drill a hole for the cylindrical core requires less distraction of the joint, reducing required exposure and potential strain on the surrounding soft tissues.

As mentioned previously, the peg 230 can have a plurality (e.g., 2 to 6) of flutes 234 that extend out radially from the cylindrical core 232. Flutes 234 provide interference fit during implantation for initial stability, resistance to rotation about the proximal-distal axis, and a large surface area for bony ingrowth or ongrowth. The cross section of each flute 234 can be generally triangular with a single sharp corner at the midpoint of the outer edge. The flutes 234 can be oriented such that they extend directly in the dorso-volar or radio-ulnar directions, or they can be oriented diagonally instead. Flutes 234 can be 0.7 to 1.0 mm thick.

The profile of each flute 234 can extend 0.8-1.2 mm away from the surface of the cylindrical core 232, creating an interference fit relative to the drilled core diameter. The flutes 234 are straight and parallel to the cylindrical core 232 of the peg 230 near the underside 203 of the main body portion 210. The flutes 234 taper slightly near the proximal tip of the peg 230, with a taper angle of 6° from the proximal-distal axis, to aid insertion of the implant into the prepared drill hole.

While the peg 230 is the primary means of fixation, the fixation region 220 can include a secondary means of fixation. More specifically, the fixation region 220 can include a plurality of spikes 240 (e.g., 2 to 4 spikes) that serve as adjuvant fixation features (See, Figs. 9A and 9B). Spikes 240 can be positioned symmetrically in the dorso-volar or radio-ulnar directions. Alternatively, they can be positioned diagonally across from the peg, maintaining radial symmetry about the proximal-distal axis (Figs. 11A-11D). Spikes 240 are positioned away from the center of the underside 203 of the main body portion 210, by 2-5 mm in the dorsal or volar directions and 3-6 mm in the radial or ulnar directions in one embodiment. Spikes 240 are positioned to maximize distance from the peg 230 while remaining within the bounds of the resection surface. This provides increased resistance to axial rotation.

Spikes 240 can be aligned parallel to an angled peg (e.g., angled peg 230) or may be perpendicular to the underside 203 of the main body portion 210 regardless of peg angle. They extend 2.0-2.5 mm from the underside 203. Spikes 240 can have a taper angle of 40°.

The underside 203 of the main body portion 210 and the outer surface of all fixation features (e.g., the peg 230 and spikes 240) can be porous to facilitate bony ingrowth for long-term fixation. These parts 203, 230, 240 can be manufactured as a porous coating layer or with additive manufacturing (Figs. 9A-9F).

In one implementation, the articulation region of the trapezial component 200 is approximately 2.5 mm thick at the center of the saddle, corresponding to a minimal resection of the trapezium to prevent /overstuffing of the joint while maintaining as much native trapezium bone stock as possible.

### Trapezial Component 400 (Figs. 13A-13F)

As mentioned previously, the trapezial component 400, like the trapezial component 200, is the second component of the prosthesis (implant) and is configured to mate with the metacarpal component 300 to form the prosthesis. The trapezial component 400 shares a number of similarities with the trapezial component 200 and therefore, the main differences are highlighted below.

Unlike the trapezial component 200 which is a single-piece, monobloc component, the trapezial component 400 is a multi-piece component (much like the metacarpal component 110). The trapezial component 400 is formed of two parts, namely, a baseplate 410 with integral fixation features 430, 440 and an insert 420 that interfaces with the baseplate 410. The insert 420 faces the CMC joint. A monobloc piece consisting of the baseplate 410 and fixation features 430, 440 is coupled to the insert 420. As described below, the fixation features 430, 440 contact and extend into the native trapezium. As with the previous embodiment, the trapezial component 400 is designed to resurface the distal saddle-shaped anatomy of the trapezium where the trapezium articulates with the first metacarpal. The trapezial component 400 is symmetric in the radio-ulnar direction such that a given component can be used for both left and right hands.

The baseplate 410 represents the bottommost portion of the main body portion of the trapezial component 400 that rests on the prepared surface of the trapezium and the insert 420 includes the articulation (articular) surface that articulates with the first metacarpal. The baseplate 410 and integral fixation features 430, 440 are formed of a suitable material, such as medical grade titanium alloy (Ti-6Al-4V) or cobalt-chrome alloy, while the insert 420 is formed of a suitable material that can be different than the material of the baseplate 410 and can be formed of UHMWPE.

As described herein below, fixation features that extend from the underside of the baseplate 410 are used to anchor the trapezial component 400 into the trapezium, and the insert 420 comprises the saddle-shaped UHMWPE insert 420 that is pre-assembled into the baseplate 410. It is designed to resurface the distal saddle-shaped anatomy of the trapezium that articulates with the first metacarpal. It will be understood that the baseplate 410 and the fixation features 430, 440 define a single, integral structure. The saddle-shaped articulation surface (articular region) 401 of the trapezial component 400 can have the same properties as the saddle-shaped articular region 201 of the trapezial component 200.

Fig. 13C shows the insert 420 coupled to the baseplate 410. Any number of traditional techniques can be used to couple and secure the insert 420 to the baseplate 410.

The proximal surface (the underside) of the baseplate 410 is planar and rests on the resection of the native trapezium bone. The footprint has a generally rectangular shape, a dorso-volar width less than the radio-ulnar width, and rounded corners, and becomes narrower in the dorso-volar direction near the center of the articulation (i.e., an hourglass shape), not inclusive of a protruding side profile (flange 425) of the trapezial component that is discussed below and is configured to accommodate a fastener (e.g., a bone screw 450 or peg) to provide additional fixation to the bone. This shape of the baseplate 410 matches the general contour of the resected surface to minimize implant overhang. There is preferably a circular cut-out 409 (Fig. 13E) along the volar edge that increases the range of motion of the CMC joint in flexion. The insert 420 matches the footprint of the baseplate 410 and has a thickness of approximately 4 mm at the center of the articulation. The insert 420 maintains a saddle shape throughout the articular region, including along the flange 425, maintaining range of motion of the CMC joint in extension.

As mentioned, there is a small flange of added material (flange 425) along the dorsal edge of the trapezial component 400 that allows for the introduction of the screw 450 for supplemental fixation without adversely impacting the articular surface. The height and position of the flange 425 minimize potential overhang of the component while providing enough material in the baseplate 410 for a locking detail in the screw hole 415.

More specifically, as shown in Figs. 13A and 13B, both the baseplate 410 and the insert 420 have a protruding side profile (flange 425) along the dorsal edge. Thus, the baseplate 410 has a first protruding body portion (first flange region 411) and the insert 420 has a second protruding body portion (second flange region 421) that overlies the first flange region 411 when the baseplate 410 and the insert 420 are attached to one another. This protruding side profile thus defines a flange area that is offset dorsally from the saddle-shaped articulation, with the same surface curvature as the articulation surface 401 and thus, the protruding side profile does not interfere with the saddle-shaped articulation surface 401.

The protruding side profile of the trapezial component 400 includes a through hole 415 that passes therethrough. The through hole 415 thus passes completely through the second flange region 421 of the insert 420 and through the first flange region 411 of the baseplate 410. As shown best in Fig. 13C, the through hole 415 is at an angle other than 90 degrees relative to a horizontal (longitudinal) axis (i.e., the underside) of the baseplate 410. In one exemplary embodiment, the through hole 415 is formed at a 30 degree angle. Since the through hole 415 is formed entirely in the protruding side profile, the through hole 415 is offset and does not interfere with the saddle-shaped articulation surface.

The device comes in multiple sizes to better match the variation in patient anatomy. The footprint can extend 15-19 mm end to end in the radio-ulnar direction and 13-14 mm end to end in the dorso-volar direction. Not including the flange for the screw, the narrow central region of the footprint is 10-11 mm. Surgeons choose the desired size of the device based on the footprint of the proximal surface. The size is chosen to maximize the footprint area on the resected surface while minimizing overhang of the implant.

As with the prior trapezial components, the trapezial component 400 includes fixation elements that are configured to fix (attach) the trapezial component 400 to the trapezium. As shown in the figures, the baseplate 410 can include a pair of spikes 430 and a peg 440 which are preferably integrally formed with the baseplate 410. The peg 440 is preferably positioned at the center of the underside of the articular surface (saddle-shaped articulation surface)) and comprises the primary fixation element of the trapezial component 400 to fix the trapezial component 400 into the trapezium. The central peg 440 can have a generally cruciform cross section with a circular center and sharp radial flutes. The central peg 440 can have a cylindrical core with a diameter of 3-4 mm, a length of 3-5 mm from the underside of the articular surface and may taper by up to 3° across the diameter getting narrower as it extends proximally away from the baseplate 410. The central peg 440 is sized to resist flexion-extension and abduction-adduction moments on the trapezial component 400 during activities of daily living.

The central peg 440 can be oriented to extend perpendicular to the underside of the articulation (i.e., the underside of the main body portion), or it can be angled by as much as 40° from the proximal-distal axis such that the peg is biased in a volar direction as it extends away from the underside of the baseplate. Angling the peg allows for easier preparation of the bone intraoperatively while maintaining resistance to bending or torsional moments applied across the CMC joint. When the peg 440 is angled, the introduction of any instrument used to drill a hole for the cylindrical core requires less distraction of the joint, reducing required exposure and potential strain on the surrounding soft tissues.

It will be appreciated that the central peg 440 and the spikes 430 can have the same constructions and same properties as the central peg 230 and spikes 240. Accordingly, at least in one embodiment, the central peg 440 can have two to four flutes that extend out radially from the cylindrical core. Flutes provide interference fit during implantation for initial stability, resistance to rotation about the proximal-distal axis, and a large surface area for bony ingrowth or ongrowth. The cross section of each flute can be generally triangular with a single sharp corner at the midpoint of the outer edge. Flutes may also have hooks or notches along the outer edge to better resist pull-out. The flutes can be oriented such that they extend directly in the dorso-volar or radio-ulnar directions, or they can be oriented diagonally instead. Flutes can be 0.7 to 1.0 mm thick in one embodiment. The profile of each flute can extend 0.5-1.2 mm away from the surface of the cylindrical core creating an interference fit relative to the drilled core diameter. Flutes may be straight and parallel to the cylindrical core of the peg. Alternatively, they may have a taper matching that of a tapered peg or a more extreme taper angle of up to 6° to insertion of the implant into the prepared drill hole.

Two to four spikes 430 are adjuvant fixation features. The spikes 430 can be positioned symmetrically in the dorso-volar or radio-ulnar directions. Alternatively, they can be positioned diagonally across from the peg, maintaining radial symmetry about the proximal-distal axis. The spikes 430 can be positioned away from the center of the underside of the articulation, by 2-3 mm in the dorsal or volar directions and 3-4 mm in the radial or ulnar directions. The spikes 430 are positioned to maximize distance from other fixation features while remaining within the bounds of the resection surface. This provides increased resistance to axial rotation. The inclusion of spikes 430 in general also increases surface area for bony ingrowth or ongrowth.

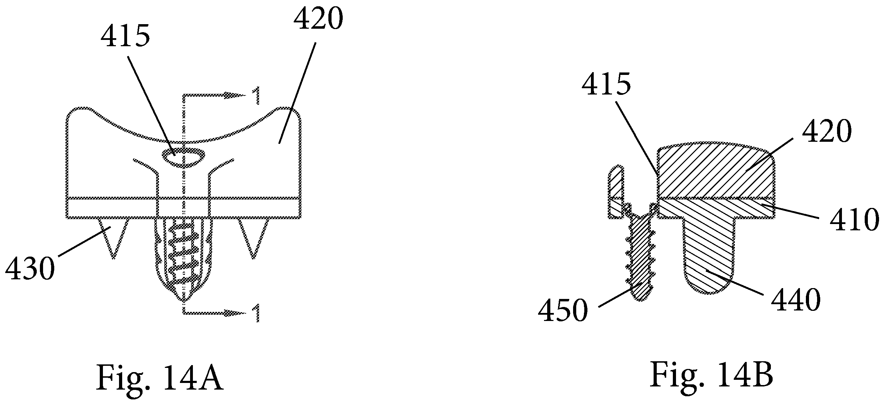

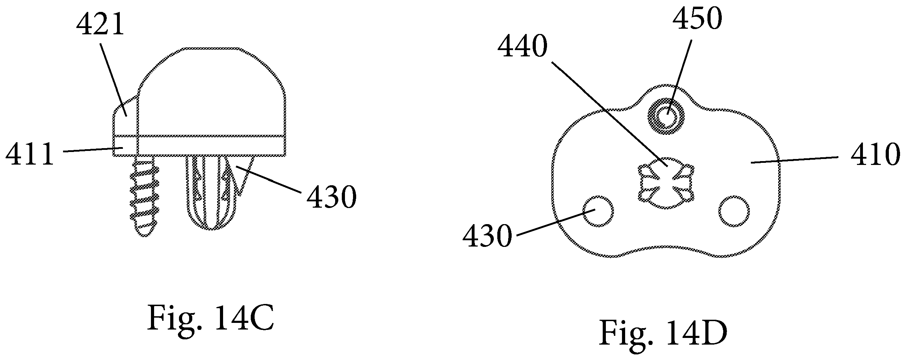

The spikes 430 can be aligned parallel to an angled peg (central peg 440) (Figs. 13A-F) or can be perpendicular to the underside of the articular surface (i.e., the underside of the main body portion) regardless of peg angle as shown in Figs. 14A-D. The spikes 430 can extend 2-3 mm from the underside of the articulation. The spikes 430 can have a taper angle of 40° or be formed at angles other than 90°.

As mentioned, the trapezial component 400 can have several different forms of supplemental fixation and in particular, can include the fastener 450 which as mentioned, can be in the form of a bone screw or can be separate headed peg that is devoid of threads. The bone is typically prepared prior to use of the fastener 450 in that before the fastener 450 is implanted, a hole needs to be created in the bone, generally with a drill, to allow the threads of the fastener to cut into bone. The bone screw is generally self-tapping and no need for a tap.

As shown, the screw 450 can be oriented parallel to the central peg 440 or it can be oriented in a non-parallel manner (diverging manner relative to the central peg 440).

In one embodiment, one bone screw 450 can be introduced through a hole in the dorsal flange of the trapezial component for supplemental fixation. The screw 450 has a core diameter of 1.5-2.5 mm and can be up to 15 mm long. Screw heads are 2.0-3.5 mm in diameter with standard Torx or hexlobe drive sizes from T5 to T7. There can be locking features on the screw head that engage with the baseplate at a fixed or variable angle to rigidly connect the screw to the baseplate. Screw threads can be standard forms or custom made for the implant. It will be appreciated that the use of the screw 450 is optional.

The pair of spikes 430 are thus stiff enough to penetrate the cancellous bone without requiring pre-drilling, along with the central peg 440 that requires bone preparation before implantation and a third supplemental fixation (e.g., the bone screw 450) that passes through the baseplate 410 into the trapezium. The bone screw 450 is configured to pass through the through hole 415 into the cancellous bone and thus, the bone screw 450 is angled into the bone (e.g., bone screw 450 can be at a 30 degree angle or other angle).

It will also be appreciated that the dimensions listed in the complete set of figures filed herewith, such as angular measurements of any of the components described herein, are only exemplary in nature and not limiting of the scope of the present disclosure.

The disclosed prosthesis 100 is thus comprised of a trapezial component and a thumb metacarpal component that replace the damaged articular surfaces of the trapezium and the thumb metacarpal bones, respectively. The prosthesis 100 thus replaces the damaged articular surfaces of the trapezium and the thumb metacarpal bones, respectively, to eliminate pain and restore the thumb's CMC joint function in terms of motion and strength.

In one embodiment, the prosthesis consists of the combination of the metacarpal component 110 and the trapezium component 200, while in another embodiment, the prosthesis consists of the combination of the metacarpal component 300 and the trapezial component 400.

The components of the proposed device have semi-congruent toroidal articular surfaces that replace the damaged articular surfaces of the native trapezium and thumb metacarpal bones. The joint surfaces of the device components can create a metal-on-polyethylene articulation and allow motions of flexion-extension, abduction-adduction, and axial rotation between components. Fixation of the prosthesis 100 is ensured by bone ingrowth on the baseplate and stem of the metacarpal component and on the baseplate and spikes of the trapezial component.

In addition, as mentioned herein, the shape of the stem 120 is purposely modeled after the implantation site and more specifically, is modeled after the inner canal (cavity) of the thumb metacarpal and is designed so that it does not suffer from the deficiencies of the conventional CMC prostheses, which were prone to loosening.

It is to be understood that like numerals in the drawings represent like elements through the several figures, and that not all components and/or steps described and illustrated with reference to the figures are required for all embodiments or arrangements.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", when used in this specification, specify the presence of stated features, integers, steps, operations, elements, and/or components, but do not preclude the presence or addition of one or more other features, integers, steps, operations, elements, components, and/or groups thereof.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

The subject matter described above is provided by way of illustration only and should not be construed as limiting. Various modifications and changes can be made to the subject matter described herein without following the example embodiments and applications illustrated and described, and without departing from the scope of the present invention, which is set forth in the following claims.

## Claims

1. A prosthesis for a carpometacarpal joint of a thumb comprising:
a metacarpal component (110, 300) having a first articular surface (153) and a stem (120) configured for insertion into a metacarpal canal of a thumb metacarpal bone to anchor the metacarpal component to the thumb metacarpal bone, the stem having a dorsal surface (130) and an opposing volar surface (132), each of the dorsal surface and the volar surface being defined by a proximal region (121) and a distal region (122), wherein the proximal region of the volar surface has a volar taper angle (αᵥ₁) that is greater than a volar taper angle (αᵥ₂) of the distal region of the volar surface and wherein a dorsal taper angle (α_{d1}) of proximal region of the dorsal surface is less than the volar taper angle of the proximal region of the volar surface; and
a trapezial component (200, 400) having a second articular surface (201) cooperating with the first articular surface, the trapezial component being configured to be coupled to a trapezium bone;
wherein the first articular surface has a first saddle shape and the second articular surface has a second saddle shape and articulates with the first articular surface;
wherein the first articular surface includes a ventral lip and a dorsal lip, **characterised in that** the first articular surface includes at least one of a first concave cut-out (151, 302) formed in the ventral lip, the first concave cut-out being configured and located to reduce impingement on the trapezium during flexing of the thumb, and a second cut-out (303) formed in the dorsal lip, the second cut-out being configured and located to reduce impingement on the trapezium during extension of the thumb.

2. The prosthesis of claim 1, wherein the trapezial component further includes a baseplate (410) and an insert (420) that is coupled to the baseplate and wherein the second
articular surface is part of the insert.

3. The prosthesis of claim 1, wherein the metacarpal component has a monobloc construction with the first articular surface being formed along a top surface of the monobloc construction.

4. The prosthesis of claim 1, wherein the volar taper angle > a radio-ulnar taper angle > the dorsal taper angle and wherein each of the volar taper angle, a radio-ulnar taper angle and the dorsal taper angle has a greater value in the proximal region of the stem compared to respective values in the distal region of the stem.

5. The prosthesis of claim 1, wherein the volar taper angle in the proximal region of the stem is between 40° and 52°, while the volar taper angle in the distal region of the stem is between 3° and 9°, wherein the radio-ulnar taper angle in the proximal region of the stem is between 8° and 24°, while the radio-ulnar taper angle in the distal region of the stem is between 4° and 6°, wherein the dorsal taper angle in the proximal region of the stem is between 3° and 6°, while the dorsal taper angle in the distal region of the stem is less than 3°.

6. The prosthesis of claim 1, wherein the stem has a non-homogenous elastic modulus, wherein the elastic modulus has a linear progression from the proximal end to the distal end, whereby the elastic modulus progressively decreases in a direction from the proximal end to the distal end.

7. The prosthesis of claim 6, wherein the elastic modulus at the proximal end is between 50 and 120 GPa, and the elastic modulus at the distal end is between 12 and 118 GPa.

8. The prosthesis of claim 1, wherein the second articular surface is formed in an insert (420) that is coupled to a baseplate (410) of the trapezial component, the articulation of the first
articular surface on the second articular surface comprising a toroid on toroid articulation.

9. The prosthesis of claim 1, wherein the first articular surface includes both the first concave cut-out formed in the ventral lip and the second cut-out formed in the dorsal lip, the first concave cut-out being formed at one end of the first articular surface, while the second cut-out being formed at an opposite end of the first articular surface.

10. The prosthesis of claim 8, wherein the first saddle shape is defined by a first radius (R1) in a radio-ulnar direction and by a second radius (R2) in a dorsal-volar direction, and the second saddle shape is defined by a third radius (R3) in the radio-ulnar direction and by a fourth radius (R4) in the dorsal-volar direction, wherein a ratio of R4/R2 is between 80% to 96% and a ratio of R1/R3 is between 80% to 96%.

11. The prosthesis of claim 1, wherein the trapezial component has an articulation region that includes the second articular surface and a fixation region (220) for contacting and
extending into the trapezium, the articulation region of the trapezial component having an underside that interfaces with the fixation region and wherein both the underside of the articulation region and at least a portion of the fixation region comprise a porous surface, the fixation region including at least one fixation peg (230) and a plurality of spikes (240)
that extend outwardly from an underside of the articulation region, wherein the at least one fixation peg is perpendicular to the underside of the articulation region.

12. The prosthesis of claim 11, wherein the at least one fixation peg has a cruciform shaped cross section with a cylindrical center (232) and radial flutes (234) that extend radially outwardly from the cylindrical center and are configured to engage bone.

13. The prosthesis of claim 1, wherein the metacarpal component is defined by a main body portion (301) having an underside from which the stem extends, the main body portion including a dorsal access cut-out (310) in the form of a recessed notch formed along a
dorsal edge of the main body portion of the metacarpal component, the dorsal access cut-out extending completely along the dorsal edge from the first articular surface to the underside of the main body portion.

14. The prosthesis of claim 2, wherein the baseplate includes a first flange portion (425) located along a dorsal edge of the baseplate and the insert includes a second flange portion (421)
located along a dorsal edge of the insert, the first and second flange portions being superimposed with respect to one another, the trapezial component having a through hole (415) formed therein, the through hole passing - through the first flange portion and the second flange portion for receiving a fastener for fixing the trapezial component to the trapezium.

15. The prosthesis of claim 14, wherein the fastener comprises a bone screw (450) and the baseplate further including an integral fixation peg (440), the through hole being formed along an axis that is either parallel to a longitudinal axis of the fixation peg or diverges from the longitudinal axis of the fixation peg.

## Patentansprüche

1. Prothese für ein Karpal-Mittelhandknochengelenk eines Daumens, umfassend:
eine Mittelhandknochenkomponente (110, 300) mit einer ersten Gelenkoberfläche (153) und einem Schaft (120), der zum Einsetzen in einen Mittelhandknochenkanal eines Daumenmittelhandknochens konfiguriert ist, um die Mittelhandknochenkomponente an dem Daumenmittelhandknochen zu verankern, wobei der Schaft eine dorsale Oberfläche (130) und eine entgegengesetzte volare Oberfläche (132) aufweist, wobei die dorsale Oberfläche und die volare Oberfläche jeweils durch einen proximalen Bereich (121) und einen distalen Bereich (122) definiert sind, wobei der proximale Bereich der volaren Oberfläche einen volaren Verjüngungswinkel (αᵥ₁) aufweist, der größer als ein volarer Verjüngungswinkel (αᵥ₂) des distalen Bereichs der volaren Oberfläche ist, und wobei ein dorsaler Verjüngungswinkel (α_{d1}) des proximalen Bereichs der dorsalen Oberfläche kleiner als der volare Verjüngungswinkel des proximalen Bereichs der volaren Oberfläche ist; und
eine Trapeziumkomponente (200, 400) mit einer zweiten Gelenkoberfläche (201), die mit der ersten Gelenkoberfläche zusammenwirkt, wobei die Trapeziumkomponente so konfiguriert ist, dass sie mit einem Trapezium-Knochen gekoppelt wird;
wobei die erste Gelenkoberfläche eine erste Sattelform aufweist und die zweite Gelenkoberfläche eine zweite Sattelform aufweist und mit der ersten Gelenkoberfläche gelenkig verbunden ist;
wobei die erste Gelenkoberfläche eine ventrale Lippe und eine dorsale Lippe einschließt, **dadurch gekennzeichnet, dass** die erste Gelenkoberfläche mindestens einen zwischen einem ersten konkaven Ausschnitt (151, 302), der in der ventralen Lippe ausgebildet ist, wobei der erste konkave Ausschnitt konfiguriert und angeordnet ist, um das Auftreffen auf das Trapezium während des Beugens des Daumens zu reduzieren, und einem zweiten Ausschnitt (303), der in der dorsalen Lippe ausgebildet ist, wobei der zweite Ausschnitt konfiguriert und angeordnet ist, um das Auftreffen auf das Trapezium während des Austreckens des Daumens zu reduzieren, einschließt.

2. Prothese nach Anspruch 1, wobei die Trapeziumkomponente ferner eine Basisplatte (410) und einen Einsatz (420) einschließt, der mit der Basisplatte gekoppelt ist, und wobei die zweite Gelenkoberfläche Teil des Einsatzes ist.

3. Prothese nach Anspruch 1, wobei die Mittelhandknochenkomponente eine Monoblockkonstruktion aufweist, wobei die erste Gelenkoberfläche entlang einer oberen Oberfläche der Monoblockkonstruktion ausgebildet ist.

4. Prothese nach Anspruch 1, wobei der volare Verjüngungswinkel > ein radio-ulnarer Verjüngungswinkel > der dorsale Verjüngungswinkel ist und wobei der volare Verjüngungswinkel, ein radio-ulnarer Verjüngungswinkel und der dorsale Verjüngungswinkel jeweils einen größeren Wert im proximalen Bereich des Schaftes im Vergleich zu jeweiligen Werten im distalen Bereich des Schaftes aufweisen.

5. Prothese nach Anspruch 1, wobei der volare Verjüngungswinkel im proximalen Bereich des Schaftes zwischen 40° und 52° liegt, während der volare Verjüngungswinkel im distalen Bereich des Schaftes zwischen 3° und 9° liegt, wobei der radio-ulnare Verjüngungswinkel im proximalen Bereich des Schaftes zwischen 8° und 24° liegt, wobei der radio-ulnare Verjüngungswinkel im distalen Bereich des Schaftes zwischen 4° und 6° liegt, wobei der dorsale Verjüngungswinkel im proximalen Bereich des Schaftes zwischen 3° und 6° liegt, während der dorsale Verjüngungswinkel im distalen Bereich des Schaftes weniger als 3° beträgt.

6. Prothese nach Anspruch 1, wobei der Schaft ein nicht-homogenes Elastizitätsmodul aufweist, wobei das Elastizitätsmodul einen linearen Verlauf von dem proximalen Ende zu dem distalen Ende aufweist, wodurch das Elastizitätsmodul in einer Richtung von dem proximalen Ende zu dem distalen Ende progressiv abnimmt.

7. Prothese nach Anspruch 6, wobei das Elastizitätsmodul am proximalen Ende zwischen 50 und 120 GPa liegt und das Elastizitätsmodul am distalen Ende zwischen 12 und 118 GPa liegt.

8. Prothese nach Anspruch 1, wobei die zweite Gelenkoberfläche in einem Einsatz (420) ausgebildet ist, der mit einer Basisplatte (410) der Trapeziumkomponente gekoppelt ist, wobei das Gelenk der ersten Gelenkoberfläche auf der zweiten Gelenkoberfläche ein toroidförmiges-auftoroidförmiges Gelenk umfasst.

9. Prothese nach Anspruch 1, wobei die erste Gelenkoberfläche sowohl den ersten konkaven Ausschnitt, der in der ventralen Lippe ausgebildet ist, als auch den zweiten Ausschnitt, der in der dorsalen Lippe ausgebildet ist, einschließt, wobei der erste konkave Ausschnitt an einem Ende der ersten Gelenkoberfläche ausgebildet ist, während der zweite Ausschnitt an einem gegenüberliegenden Ende der ersten Gelenkoberfläche ausgebildet ist.

10. Prothese nach Anspruch 8, wobei die erste Sattelform durch einen ersten Radius (R1) in einer radio-ulnaren Richtung und durch einen zweiten Radius (R2) in einer dorsal-volaren Richtung definiert ist und die zweite Sattelform durch einen dritten Radius (R3) in der radio-ulnaren Richtung und durch einen vierten Radius (R4) in der dorsal-volaren Richtung definiert ist, wobei ein Verhältnis von R4/R2 zwischen 80 % und 96 % liegt und ein Verhältnis von R1/R3 zwischen 80 % und 96 % liegt.

11. Prothese nach Anspruch 1, wobei die Trapeziumkomponente einen Gelenkbereich aufweist, der die zweite Gelenkoberfläche und einen Fixationsbereich (220) zum Kontaktieren und Erstrecken in das Trapezium einschließt, wobei der Gelenkbereich der Trapeziumkomponente eine Unterseite aufweist, die mit dem Fixationsbereich zusammenwirkt, und wobei sowohl die Unterseite des Gelenkbereichs als auch mindestens ein Abschnitt des Fixationsbereichs eine poröse Oberfläche umfassen, wobei der Fixationsbereich mindestens einen Fixationszapfen (230) und eine Vielzahl von Dornen (240) einschließt, die sich von einer Unterseite des Gelenkbereichs nach außen erstrecken, wobei der mindestens eine Fixationszapfen senkrecht zu der Unterseite des Gelenkbereichs ist.

12. Prothese nach Anspruch 11, wobei der mindestens eine Fixationszapfen einen kreuzförmigen Querschnitt mit einer zylindrischen Mitte (232) und radialen Rillen (234) aufweist, die sich von der zylindrischen Mitte radial nach außen erstrecken und konfiguriert sind, um in den Knochen einzugreifen.

13. Prothese nach Anspruch 1, wobei die Mittelhandknochenkomponente durch einen Hauptkörperabschnitt (301) definiert ist, der eine Unterseite aufweist, von der sich der Schaft erstreckt, wobei der Hauptkörperabschnitt einen dorsalen Zugangsausschnitt (310) in Form einer vertieften Kerbe einschließt, die entlang einer dorsalen Kante des Hauptkörperabschnitts der Mittelhandknochenkomponente ausgebildet ist, wobei sich der dorsale Zugangsausschnitt vollständig entlang der dorsalen Kante von der ersten Gelenkoberfläche zur Unterseite des Hauptkörperabschnitts erstreckt.

14. Prothese nach Anspruch 2, wobei die Basisplatte einen ersten Flanschabschnitt (425) einschließt, der entlang einer dorsalen Kante der Basisplatte angeordnet ist, und der Einsatz einen zweiten Flanschabschnitt (421) einschließt, der entlang einer dorsalen Kante des Einsatzes angeordnet ist, wobei der erste und der zweite Flanschabschnitt in Bezug aufeinander übereinander angeordnet sind, wobei die Trapeziumkomponente ein darin ausgebildetes Durchgangsloch (415) aufweist, wobei das Durchgangsloch durch den ersten Flanschabschnitt und den zweiten Flanschabschnitt hindurchläuft, um ein Fixationselement zur Fixation der TrapeziumKomponente an dem Trapezium aufzunehmen.

15. Prothese nach Anspruch 14, wobei das Fixationselement eine Knochenschraube (450) umfasst und die Basisplatte ferner einen integralen Fixationszapfen (440) einschließt, wobei das Durchgangsloch entlang einer Achse ausgebildet ist, die entweder parallel zu einer Längsachse des Fixationszapfens ist oder von der Längsachse des Fixationszapfens divergiert.

## Revendications

1. Prothèse pour une articulation carpo-métacarpienne d'un pouce comprenant :
un composant métacarpien (110, 300) présentant une première surface articulaire (153) et une tige (120) configurée l'insertion dans un canal métacarpien d'un os métacarpien du pouce pour ancrer le composant métacarpien à l'os métacarpien du pouce, la tige présentant une surface dorsale (130) et une surface palmaire opposée (132), la surface dorsale et la surface palmaire étant définies par une région proximale (121) et une région distale (122), la région proximale de la surface palmaire présentant un angle de conicité palmaire (αᵥ₁) étant supérieur à l'angle de conicité palmaire (αᵥ₂) de la région distale de la surface palmaire et un angle de conicité dorsale (α_{d1}) de la région proximale de la surface dorsale étant inférieur à l'angle de conicité palmaire de la région proximale de la surface palmaire ; et
un composant trapézoïdal (200, 400) présentant une seconde surface articulaire (201) coopérant avec la première surface articulaire, le composant trapézoïdal étant configuré pour être couplé à un trapèze ;
la première surface articulaire ayant une première forme de selle et la seconde surface articulaire ayant une seconde forme de selle et s'articule avec la première surface articulaire ;
la première surface articulaire comprenant une lèvre ventrale et une lèvre dorsale, **caractérisée en ce que** la première surface articulaire comprend au moins une première découpe concave (151, 302) formée dans la lèvre ventrale, la première découpe concave étant configurée et située pour réduire le pincement sur le trapèze lors de la flexion du pouce, et une seconde découpe (303) formée dans la lèvre dorsale, la seconde découpe étant configurée et située pour réduire le pincement sur le trapèze lors de l'extension du pouce.

2. Prothèse selon la revendication 1, le composant trapézoïdal comprenant en outre une plaque de base (410) et un insert (420) étant couplé à la plaque de base et où la seconde surface articulaire fait partie de l'insert.

3. Prothèse selon la revendication 1, le composant métacarpien ayant une construction monobloc avec la première surface articulaire étant formée le long d'une surface supérieure de la construction monobloc.

4. Prothèse selon la revendication 1, où l'angle de conicité palmaire > à un angle de conicité radio-ulnaire > à l'angle de conicité dorsale et où l'angle de conicité palmaire, un angle de conicité radio-ulnaire et l'angle de conicité dorsale ont une valeur plus grande dans la région proximale de la tige comparée aux valeurs respectives dans la région distale de la tige.

5. Prothèse selon la revendication 1, l'angle de conicité palmaire dans la région proximale de la tige étant compris entre 40 et 52°, tandis que l'angle de conicité palmaire dans la région distale de la tige est compris entre 3 et 9°, l'angle de conicité radio-ulnaire dans la région proximale de la tige étant compris entre 8 et 24°, tandis que l'angle de conicité radio-ulnaire dans la région distale de la tige est compris entre 4 et 6°, l'angle de conicité dorsale dans la région proximale de la tige étant compris entre 3 et 6°, tandis que l'angle de conicité dorsale dans la région distale de la tige est inférieur à 3°.

6. Prothèse selon la revendication 1, la tige comportant un module élastique non homogène, le module élastique présentant une progression linéaire de l'extrémité proximale à l'extrémité distale, ce par quoi le module élastique diminue progressivement dans une direction allant de l'extrémité proximale à l'extrémité distale.

7. Prothèse selon la revendication 6, le module élastique à l'extrémité proximale étant compris entre 50 et 120 GPa, et le module élastique à l'extrémité distale est compris entre 12 et 118 GPa.

8. Prothèse selon la revendication 1, la seconde surface articulaire étant formée dans un insert (420) qui est couplé à une plaque de base (410) du composant trapézoïdal, l'articulation de la première surface articulaire sur la seconde surface articulaire comprenant une articulation toroïde sur toroïde.

9. Prothèse selon la revendication 1, la première surface articulaire comprenant à la fois la première découpe concave formée dans la lèvre ventrale et la seconde découpe formée dans la lèvre dorsale, la première découpe concave étant formée à une extrémité de la première surface articulaire, tandis que la seconde découpe étant formée à une extrémité opposée de la première surface articulaire.

10. Prothèse selon la revendication 8, la première forme de selle étant définie par un premier rayon (R1) dans la direction radio-ulnaire et par un deuxième rayon (R2) dans la direction dorso-palmaire, et la seconde forme de selle est définie par un troisième rayon (R3) dans la direction radio-ulnaire et par un quatrième rayon (R4) dans la direction dorso-palmaire, un rapport R4/R2 étant compris entre 80 et 96 % et un rapport R1/R3 étant compris entre 80 et 96 %.

11. Prothèse selon la revendication 1, le composant trapézoïdal comportant une région d'articulation qui comprend la seconde surface articulaire et une région de fixation (220) pour se mettre en contact et s'étendre dans le trapèze, la région d'articulation du composant trapézoïdal comportant un côté inférieur qui fait l'interface avec la région de fixation et où le côté inférieur de la région d'articulation et au moins une partie de la région de fixation comprennent une surface poreuse, la zone de fixation comprenant au moins une cheville de fixation (230) et une pluralité de pointes (240) qui s'étendent vers l'extérieur à partir d'un côté inférieur de la zone d'articulation, l'au moins une cheville de fixation étant perpendiculaire au côté inférieur de la zone d'articulation.

12. Prothèse selon la revendication 11, l'au moins une cheville de fixation présentant une section transversale cruciforme avec un centre cylindrique (232) et des cannelures radiales (234) qui s'étendent radialement vers l'extérieur à partir du centre cylindrique et sont configurées pour se mettre en prise avec l'os.

13. Prothèse selon la revendication 1, le composant métacarpien étant défini par une partie de corps principal (301) comportant un côté inférieur à partir duquel la tige s'étend, la partie de corps principal comprenant une découpe d'accès dorsale (310) sous la forme d'une encoche évidée formée le long d'un bord dorsal de la partie de corps principal du composant métacarpien, la découpe d'accès dorsale s'étendant complètement le long du bord dorsal de la première surface articulaire au côté inférieur de la partie de corps principal.

14. Prothèse selon la revendication 2, la plaque de base comprenant une première partie de bride (425) située le long d'un bord dorsal de la plaque de base et l'insert comprenant une seconde partie de bride (421) située le long d'un bord dorsal de l'insert, la première et la seconde partie de bride étant superposées l'une par rapport à l'autre, le composant trapézoïdal comportant un trou traversant (415) formé en son sein, le trou traversant passant à travers la première partie de bride et la seconde partie de bride pour recevoir un élément de fixation destiné à fixer le composant trapézoïdal sur le trapèze.

15. Prothèse selon la revendication 14, l'élément de fixation comprenant une vis à os (450) et la plaque de base comprenant en outre une cheville à fixation intégrale (440), le trou traversant étant formé le long d'un axe qui est soit parallèle à un axe longitudinal de la cheville de fixation, soit divergent de l'axe longitudinal de la cheville de fixation.
